(19) Europäisches Patentamt
European Patent Office
Office européen des brevets

(11) Publication number: **0 138 417**
**B1**

(12) # EUROPEAN PATENT SPECIFICATION

(45) Date of publication of patent specification: **14.03.90**

(51) Int. Cl.$^5$: **C 07 D 471/04,** A 61 K 31/505

(21) Application number: **84306434.6**

(22) Date of filing: **20.09.84**

(54) **2-substituted pyrimidohexahydroquinolines.**

(30) Priority: **26.09.83 US 535518**

(43) Date of publication of application:
**24.04.85 Bulletin 85/17**

(45) Publication of the grant of the patent:
**14.03.90 Bulletin 90/11**

(84) Designated Contracting States:
**BE CH DE FR GB IT LI LU NL SE**

(56) References cited:
**GB-A- 895 422**
**US-A-2 794 020**
**US-A-4 198 415**

(73) Proprietor: **ELI LILLY AND COMPANY**
**307, East McCarty Street**
**Indianapolis Indiana 46285 (US)**

(72) Inventor: **Nichols, Cynthia Lynn**
**6106 Norwaldo Avenue**
**Indianapolis Indiana 46220 (US)**
Inventor: **Kornfeld, Edmund Carl**
**5159 East 76th Court**
**Indianapolis Indiana 46250 (US)**
Inventor: **Schaus, John Mehnert**
**5427 North Delawara Street**
**Indianapolis Indiana 46220 (US)**

(74) Representative: **Tapping, Kenneth George et al**
**Lilly Industries Limited Patent Department**
**Erl Wood Manor**
**Windlesham Surrey, GU20 6PH (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention concerns a class of novel 2-substituted-pyrimidohexahydroquinoline derivatives and intermediates, which have been discovered to be UV absorptors for use as sunscreen agents.

Sunscreen agents are widely sold. The purpose of these agents is to absorb the UV—B radiation (approximately 280 to 320 mμ range) and allow the UV—A radiation (approximately 320 to 400 mμ range) to tan the skin. UV—B is very efficient at causing skin redness associated with sunburns, otherwise known as erythema.

The most commonly used sunscreen agents are synthetic organic compounds which usually incorporate a benzene ring in their structure. One of the oldest and still widely used sunscreen agents is *p*-aminobenzoic acid (PABA). Other commonly used agents are PABA esters, benzophenones, and derivatives of cinnamic acid or salicylic acid.

It has now been discovered that 2-substituted pyrimidohexahydroquinoline derivatives absorb UV—B radiation. This is surprising as the pyrazoloquinoline derivatives described in US—A—4,198,415 are not useful as sunscreen agents. For example, trans-dl-5-n-propyl-4,4a,5,6,7,8,8a,9-octahydro-1H (and 2H)-pyrazolo[3,4-g]quinoline has a $\lambda_{max}$=221 mμ (ε=4,400)(methanol).

A 2-aminooctahydro-6-propylpirimidoquinoline is described in EP—A—139393.

This invention concerns trans-(±)-octahydropyramido[4,5-g]quinolines of the formula (I)

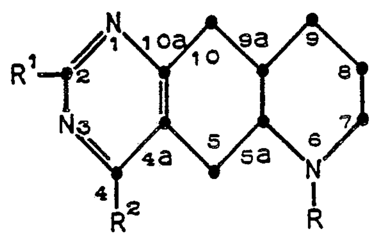

I

or a pharmaceutically acceptable salt thereof; wherein: R is $C_1$—$C_3$ alkyl or allyl; $R^1$ is OH, and $R^2$ is H or $R^1$=$R^2$=$NH_2$.

This invention also concerns the corresponding trans-(−)-enantiomer of the compounds of formula (I) having the formula (II)

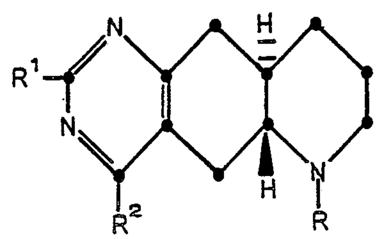

II

or a pharmaceutically acceptable salt thereof; wherein R, $R^1$ and $R^2$ are defined as above.

Preferred subgroups of compounds represented by formula (I) or (II) above include:

(a) compounds in which R is H or $C_1$—$C_3$ alkyl;

(b) compounds in which R is n-propyl; and

(c) compounds in which $R^1$ is OH and $R^2$ is H.

The compounds represented by formula (I) or (II) absorb ultraviolet light with absorption peaks in the range 265—350 mμ, (which range encompasses the UV—B — 280—320 mμ range —) and are therefore useful as sunscreen agents. The compounds are also useful as intermediates.

The compounds of formula (I) are prepared by

a) hydrolyzing of a compound of the formula (III)

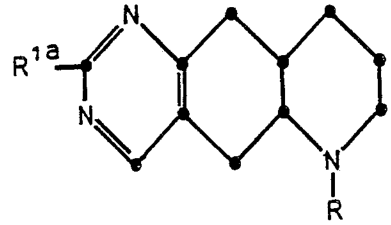

III

wherein $R^{1A}$ is S—$C_1$—$C_3$ alkyl, and R is defined as above, with an acid to provide the compounds of formula (I) wherein $R^1$ is OH and $R^2$ is H; or

b) cyclizing a compound of the formula (IV)

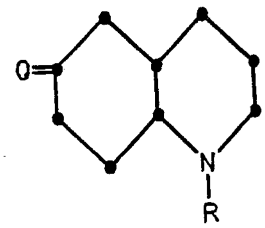

IV

wherein R is defined as above, with a compound of the formula (V)

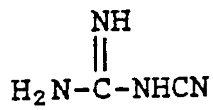

V

to provide the compounds of formula I wherein $R^1$ and $R^2$ are $NH_2$; or

c) optionally salifying the compounds of formula (I) by conventional methods.

In all the above process steps, the corresponding trans-(−)-enantiomer can be used.

The present invention also includes a cosmetic formulation having as active ingredient a compound of formula (I) or (II), or a pharmaceutically acceptable salt thereof, associated with one or more physiologically acceptable carriers or excipients therefor.

Pharmaceutically-acceptable salts of the compounds of formula (I) or (II) include salts derived from non-toxic inorganic acids such as hydrochloric acid, nitric acid, phosphoric acid, sulfuric acid, hydrobromic acid, hydriodic acid, phosphorous acid and the like, as well as salts derived from non-toxic organic acids such as aliphatic mono and discarboxylic acids, phenyl-substituted alkanoic acids, hydroxy alkanoic and alkandioic acids, aromatic acids, aliphatic and aromatic sulfonic acids. Such pharmaceutically acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, tartrate, methanesulfonate, propanesulfonate, naphthalene-1-sulfonate and naphthalene-2-sulfonate salts.

Compounds of formula (I) or (II) wherein $R^2$ is H and $R^1$ is OH are prepared by acidic hydrolysis of a trans-(±) or trans-(−)-2-$C_1$—$C_3$ alkylthio-6-$C_1$—$C_3$ alkyl or alkyl-5,5a,6,7,8,9,9a,10-octahydropyrimido-[4,5-g]quinoline. Suitable acids, which can also be used as the solvent, are mineral acids such as hydrochloric acid, sulfonic acid, phosphoric acid, and hydrobromic acid. The reaction is usually run at from room temperature to reflux temperature.

Compounds of formula (I) or (II) in which both $R^1$ and $R^2$ are $NH_2$ are prepared by cyclizing the usual trans-(±) or trans-(−)-1-substituted-6-oxodecahydroquinoline with cyanoguanidine in a high boiling mutual inert solvent such as diethyleneglycol monomethyl ether and CARBITOL (diethyleneglycol monoethyl ether) — see Modest et al., *J. Org. Chem., 30,* 1837 (1965). The reaction is run at a temperature from 170°C to reflux temperature.

While compounds of formula (I) or (II) in which R is $C_1$—$C_3$ alkyl or allyl are the primary products of this invention, those compounds of formula (I) or (II) in which R is H or CN are not only UV absorbers but are also intermediates for the preparation of compounds carrying a different alkyl group (from that of the 6-oxodecahydroquinoline starting material) or an allyl group. For example, if it is desired to prepare a compound of formula (I) or (II) in which R is allyl, it is possible (depending on the nature of the $R^1$ and $R^2$ substituents) to replace the R alkyl group with CN (using CNBr), and then to remove the CN group by hydrolysis to yield compounds wherein R is H. Suitable reagents to remove the CN group are zinc and acetic acid, zinc dust and hydrochloric acid, aqueous mineral acid such as hydrochloric acid and sulfuric acid, and hydrogenation by conventional procedures. The reaction is conducted at a temperature of from about 50°C to reflux temperature. This secondary amine derivative can then be allylated as with allyl chloride or can be reductively alkylated with an aldehyde or can be alkylated with a $C_1$—$C_3$ alkyl halide to yield a compound of formula (I) or (II) having an allyl group or a different alkyl at N—6. Incidentally, it is not necessary to replace an N-methyl with a different alkyl group in order for the above process to be useful. For example, it is possible to replace a $CH_3$ group with a $^{13}CH_3$ containing an isotopically-tagged carbon. Preferably, however, because of the reactivity of OH groups at C—2 in formula (I) or (II) to CNBr, the interchange of groups on the quinoline nitrogen should take place prior to ring closure, i.e., at the 6-oxodecahydroquinoline stage. In this procedure, a trans-(±) or trans-(−)-1-methyl-6-oxodecahydroquinoline, for example, is transformed to the 1-cyano compound — see Bach et al., *J. Med. Chem., 23,* 481

(1980) or US—A—4,198,415. Acidic hydrolysis or hydrogenolysis of this compound yields trans-(±) or trans-(−)-6-oxodecahydroquinoline. This secondary amine can then be allylated or realkylated possibly with a tagged alkyl, using allyl or alkyl halides to prepare the desired intermediate trans-(±) or trans-(−)-1-allyl or alkyl-6-oxodecahydroquinoline. The above procedures are clearly extremely useful in preparing the difficult-to-obtain N-allyl derivatives because the best procedures for obtaining the 2-alkyl-6-oxodecahydroquinolines involves multiple reduction or hydrogenation procedures, and an allyl group would not survive all of these synthetic steps.

The following specific examples more fully illustrate the preparation aspects of this invention.

Example 1

Preparation of trans-(±)-2-hydroxy-6-n-propyl-5,5a,6,7,8,9,9a,10-octahydropyrimido[4,5-g]quinoline

A mixture of 0.93 g of trans-(±)-2-methylthio-6-n-propyl-5,5a,6,7,8,9,9a,10-octahydropyrimido-[4,5-g]quinoline and 2 ml of 12N aqueous hydrochloric acid was heated to reflux temperature for 1.5 hours. The reaction mixture was then concentrated *in vacuo*. Methanol was added and the reaction mixture again concentrated *in vacuo*. A residual green oil, comprising trans-(±)-2-hydroxy-6-n-propyl-5,5a,6,7,8,9,9a,10-octahydropyrimido[4,5-g]quinoline dihydrochloride formed in the above reaction was dissolved in a mixture of ethyl acetate and ethanol. As the green oil dissolved, crystals formed. Trans-(±)-2-hydroxy-6-n-propyl-5,5a,6,7,8,9,9a,10-octahydropyrimido[4,5-g]quinoline dihydrochloride thus prepared had the following physical characteristics. $R_f$(methanol)=0.5; molecular ion at 247; yield=0.62 g. UV $\lambda_{max}$=315 mμ (ε=4,500) (95% ethanol).

| | | | |
|---|---|---|---|
| Analysis Calculated: | C, 52.50: | H, 7.24; | N, 13.12; |
| Found: | C, 52.29; | H, 7.08; | N, 12.88. |

Example 2

Preparation of trans-(±)-2,4-diamino-6-n-propyl-5,5a,6,7,8,9,9a,10-octahydropyrimido[4,5-g]quinoline

A reaction mixture was prepared from 5 g of trans-(±)-1-n-propyl-6-oxodecahydroquinoline and 3.24 g of cyanoguanidine in 12.5 ml of diethyleneglycol monoethylether (CARBITOL). The reaction mixture was heated to reflux temperature under an $N_2$ atmosphere for about 6 hours, and was then poured into a mixture of very dilute aqueous NaOH and ethyl acetate. A tan solid precipitated which was collected by filtration. Recrystallization of the filter cake from a methanol-ethyl acetate solvent mixture yielded 1.50 g (22% yield) of a white powder melting at 238—240°C with decomposition. Trans-(±)-2,4-diamino-6-n-propyl-5,5a,6,7,8,9,9a,10-octahydropyrimido[4,5-g]quinoline thus prepared had the following physical characteristics: Infrared spectrum, at 3385, 3163, 1630, 1593, 1569, 1441 Ultraviolet spectrum (methanol); peaks at 201, 212, 228, 283 UV $\lambda_{max}$=283 mμ (ε=6,300) (methanol).

Mass spectrum: peaks at 261, 218, 138, 125, 124, 96.

| | | | |
|---|---|---|---|
| Analysis Calculated: | C, 64.33: | H, 8.87; | N, 26.80; |
| Found: | C, 64.32; | H, 8.75; | N, 27.03. |

A dihydrochloride salt was prepared and recrystallized from methanol/ethyl acetate to yield a white powder melting above 250°C. The salt had the following elemental analysis.

| | | | | |
|---|---|---|---|---|
| Analysis Calculated: | C, 50.30: | H, 7.54; | N, 20.95; | Cl, 20.21; |
| Found: | C, 50.03; | H, 7.34; | N, 20.67; | Cl, 21.32. |

Example 3

Preparation of trans-(±)-2,4-diamino-5,5a,6,7,8,9,9a,10-octahydropyrimido[4,5-g]quinoline

A mixture of 29 mg of trans-(±)-6-cyano-2,4-diamino-5,5a,6,7,8,9,9a,10-octahdyropyrimido[4,5-g]quinoline and 9 ml of 6N aqueous hydrochloric acid was heated to reflux temperature for about four hours. The reaction was concentrated to yield 49 mg of a white solid. The solid was dissolved in water and the aqueous solution made basic with NaOH. The alkaline mixture was extracted with 1:3 isopropanol chloroform, then the extract was concentrated to give 20 mg of a white solid. The solid was chromatographed on a 10 mm × 5 cm flash $SiO_2$ column using $CH_3OH$ with a trace of $NH_4OH$ as eluant. Fraction 10 yielded a white solid which was recrystallized from methanol:ethyl acetate to yield 8 mg of a white solid, trans-(±)-2,4-diamino-5,5a,6,7,8,9,9a,10-octahydropyrimido[4,5-g]quinoline, m.p.=259—260°C (decomposition).

In addition to their use as ultraviolet light absorbers, the compounds of formula I also serve as intermediates. Examples of such use are contained in the above synthetic procedures; i.e., the 2—OH derivative is prepared from a 2-methylthio compound or a 2-methoxy derivative by methylation of a 2-hydroxy derivative. Additionally, a 2-hydroxy group can be replaced directly with dimethylamine or monomethylamine to yield trans-(±) or trans-(−)-2-dimethylamine or methylamino-6-alkyl or allyl-octahydropyrimido[4,5-g]quinoline. Additionally, the 2—OH group can be reacted directly, or via a 2-chloro group, with ammonia or sodamide to yield a 2-amino derivative.

In employing the compounds of formula I as ultraviolet light absorbers or sunscreen agents, a compound according to formula I is incorporated into a cream base having the following ingredients:

CREAM BASE I

| Ingredient | % by Weight |
| --- | --- |
| Cetyl alcohol | 3.5 |
| Lanolin | 3.5 |
| Polysorbate 60 (1) | 4.0 |
| Falba absorption base (2) | 0.5 |
| Carbopol 934 (3) | 0.06 |
| Sorbitan monostearate | 2.6 |
| Anhydrous citric acid | 0.07 |
| Methyl paraben | 0.2 |
| Propyl paraben | 0.15 |
| Tenox IV (4) | |
| Isopropyl myristate | 12 |
| Cloroicil 200 (5) | 0.2 |
| 98% triethanolamine | 0.17 |
| Sunscreen agent | up to 5.0 |
| Fragrance | 0 - 0.3 |
| Water q.s. to | 100 |

(1) polyoxyethylene (20) sorbitan mono-oleate.

(2) a mixture of mineral oil, lanolin, lanolin alcohol, paraffin and beeswax.

(3) a cross-linked acrylic acid polymer.

(4) a mixture of corn oil, BHT-(2,6-di-t-butyl-6-methylphenol) and BHA (t-butyl-p-methoxyphenol).

(5) cis-isomer of 1-(3-chloroallyl)-3,5,7-triaza-1-azonia adamantane chloride.

CREAM BASE II

| Ingredient | % by Weight |
|---|---|
| Isopropylmyristole | 13.5 |
| Mineral oil | 8.00 |
| 70% Sorbitol solution | 7.50 |
| Triglyceryldiisosterate | 5.50 |
| Beeswax | 4.00 |
| Polyethylene | 4.00 |
| Bentone #38(1) | 1.5 |
| Imidazolinylurea | 0.40 |
| Sodium tetraborate | 0.38 |
| Propylparaben | 0.20 |
| Methylparaben | 0.20 |
| Fragrance material | 0 - .30 |
| Sunscreen agent | 1 - 5 |
| Water q.s. to | 100 |

(1) Reaction product of bentonite and a quaternary ammonium halide $[(CH_3)_2R_2N]^+Cl^-$ where R is a tallow fatty radical

**Claims**

1. A trans-(±) compound of the formula (I)

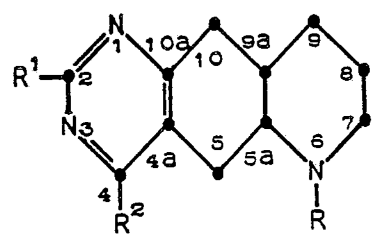

I

or a pharmaceutically acceptable salt thereof; wherein: R is $C_1$—$C_3$ alkyl or allyl; $R^1$ is OH, and $R^2$ is H or $R^1$=$R^2$=$NH_2$.

2. A trans-(−)- compound of the formula (II)

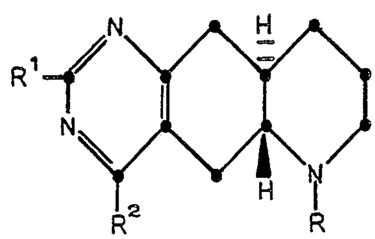

II

or a pharmaceutically acceptable salt thereof, wherein R, $R^1$ and $R^2$ are defined as in claim 1.

3. A compound of claim 1 or 2 wherein R is $C_1$—$C_3$ alkyl.

4. A compound of claim 3 in which R is n-propyl.

5. A compound of claim 1 or 2 in which $R^1$ is OH and $R^2$ is H.

6. Any one of the following compounds or a pharmaceutically acceptable salt thereof:
trans-(±)-2,4-diamino-6-n-propyl-5,5a,6,7,8,9,9a,10-octahydropyrimido[4,5-g]quinoline
trans-(±)-2-hydroxy-6-n-propyl-5,5a,6,7,8,9,9a,10-octahydropyrimido[4,5-g]quinoline
trans-(±)-2,4-diamino-5,5a,6,7,8,9,9a,10-octahydropyrimido[4,5-g]quinoline.

7. A process for preparing a compound of formula (I) or (II), or a pharmaceutically acceptable salt thereof, as claimed in any one of claims 1 to 7, which comprises:

a) hydrolyzing a compound of the formula (III)

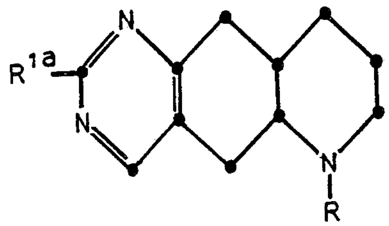

III

wherein $R^{1a}$ is $C_1$—$C_3$ alkyl, and R is defined as in claim 1) with an acid to provide the compounds of formula (I) wherein $R^1$ is OH and $R^2$ is H; or

b) cyclizing a compound of the formula (IV)

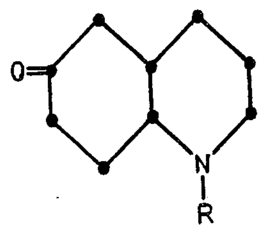

IV

wherein R is defined as in claim 1, with a compound of the formula (V)

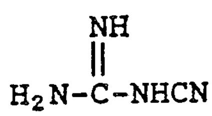

V

to provide the compounds of formula I wherein $R^1$ and $R^2$ are $NH_2$; or

c) optionally salifying the compounds of formula (I) by conventional methods.

8. A cosmetic formulation which comprises as active ingredient a compound of formula (I) or (II), or a pharmaceutically acceptable salt thereof, as claimed in any one of claims 1 to 7, associated with one or more physiologically acceptable carriers or excipients therefor.

**Patentansprüche**

1. Trans-(±)-verbindung der Formel (I)

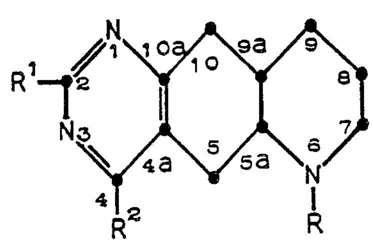

I

oder ein pharmazeutisch annehmbares Salz hiervon, worin R für $C_1$—$C_3$-Alkyl oder Allyl steht, $R^1$ für OH steht und $R^2$ für H steht oder $R^1$ gleich $R^2$ ist und für $NH_2$ steht.

2. Trans-(−)-verbindung der Formel (II)

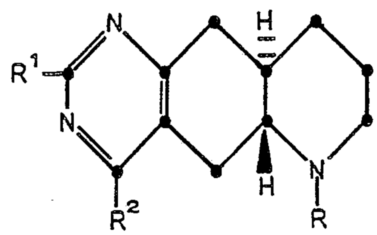

II

oder ein pharmazeutisch annehmbares Salz hiervon, worin R, $R^1$ und $R^2$ wie im Anspruch 1 definiert sind.

3. Verbindung nach Anspruch 1 oder 2, dadurch gekennzeichnet, daß R für $C_1$—$C_3$-Alkyl steht.

4. Verbindung nach Anspruch 3, dadurch gekennzeichnet, daß R für n-Propyl steht.

5. Verbindung nach Anspruch 1 oder 2, dadurch gekennzeichnet, daß $R^1$ für OH steht und $R^2$ für H steht.

6. Irgendeine der folgenden Vebindungen oder ein pharmazeutische annehnmbares Salz hiervon:
trans-(±)-2,4-Diamino-6-n-propyl-5,5a,6,7,8,9,9a,10-octahydropyrimido[4,5-g]chinolin,
trans-(±)-2-Hydroxy-6-n-propyl-5,5a,6,7,8,9,9a,10-octahydropyrimido[4,5-g]chinolin oder
trans-(±)-2,4-Diamino-5,5a,6,7,8,9,9a,10-octahydropyrimido[4,5-g]chinolin.

7. Verfahren zur Herstellung einer Verbindung der Formeln (I) oder (II) oder eines pharmazeutisch annehmbaren Salzes hiervon gemäß einem der Ansprüche 1 bis 6, dadurch gekennzeichnet, daß

(a) eine Verbindung der Formel (III)

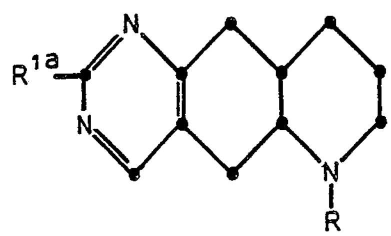

III

worin $R^{1a}$ für S-$C_1$—$C_3$-Alkyl steht und R wie im Anspruch 1 definiert ist, durch Hydrolyse mit einer Säure in eine Verbindung der Formel (I) überführt wird, worin $R^1$ für OH steht und $R^2$ für H steht, oder

(b) eine Verbindung der Formel (IV)

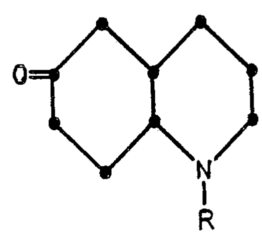

IV

worin R wie im Anspruch 1 definiert ist, durch Cyclisierung mit einer Verbindung der Formel (V)

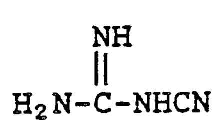

V

in einer Verbindung der Formel (I) überführt wird, worin $R^1$ und $R^2$ jeweils $NH_2$ sind, und

(c) die erhaltenen Verbindungen der Formel (I) unter Anwendung herkömmlicher Verfahren gegebenenfalls in Salze überführt werden.

8. Kosmetische Formulierung, dadurch gekennzeichnet, daß sie eine Verbindung der Formeln (I) oder (II) oder ein pharmazeutisch annehmbares Salz hiervon nach einem der Ansprüche 1 bis 7 als Wirkstoff zusammen mit einem oder mehreren physiologisch annehmbaren Trägern oder Hilfsstoffen hierfür enthält.

**Revendications**

1. Composé trans-(±) de formule (I)

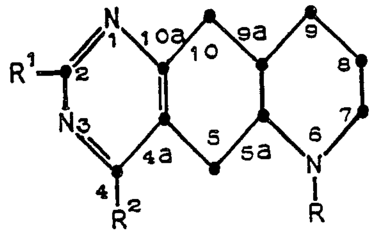

I

ou un de ses sels pharmaceutiquement acceptables; dans lequel R représente un groupe alkyle en $C_1$—$C_3$ ou un groupe allyle; $R^1$ représente OH et $R^2$ représente H ou bien $R^1=R^2=NH_2$.

2. Composé trans-(−) de formule (II)

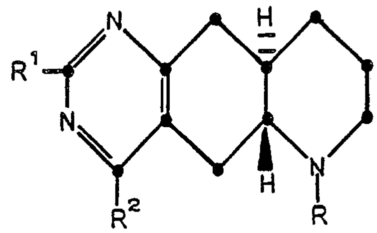

II

ou un de ses sels pharmaceutiquement acceptables; dans lequel R, $R^1$ et $R^2$ ont les significations définies dans la revendication 1.

3. Composé selon la revendication 1 ou 2 dans lequel R représente un groupe alkyle en $C_1$—$C_3$.

4. Composé selon la revendication 3 dans lequel R représente un groupe n-propyle.

5. Composé selon la revendication 1 ou 2 dans lequel $R^1$ représente OH et $R^2$ représente H.

6. L'un quelconque des composés ci-après ou un de leurs sels pharmaceutiquement acceptables:
la trans-(±)-2,4-diamino-6-n-propyl-5,5a,6,7,8,9,9a,10-octahydropyrimido[4,5-g]quinoléine
la trans-(±)-2-hydroxy-6-n-propyl-5,5a,6,7,8,9,9a,10-octahydropyrimido[4,5-g]quinoléine
la trans-(±)-2,4-diamino-5,5a,6,7,8,9,9a,10-octahydropyrimido[4,5-g]quinoléine.

7. Procédé pour la préparation d'un composé de formule (I) ou (II), ou d'un de ses sels pharmaceutiquement acceptables, selon l'une quelconque les revendications 1 à 7, de procédé consistant à:

a) hydrolyser un composé de formule (III)

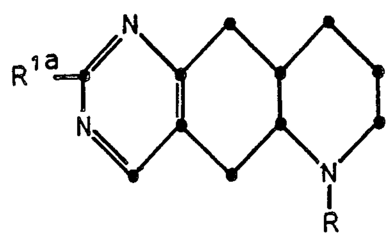

III

dans lequel $R^{1a}$ représente un groupe S-alkyle en $C_1$—$C_3$, tandis que R a la signification telle que définie dans la revendication 1; avec un acide pour obtenir les composés de formule (I) dans lesquels $R^1$ représente OH et $R^2$ représente H; ou

b) cycliser un composé de formule (IV)

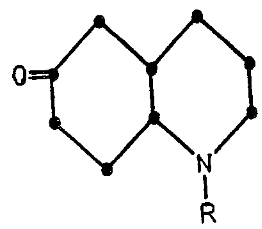

IV

dans lequel R a la signification définis dans la revendication 1, avec un composé de formule (V)

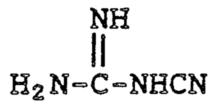

V

pour obtenir les composés de formule (I) dans lesquels $R^1$ et $R^2$ représentant $NH_2$; ou

c) éventuellement salifier les composés de formule (I) via des procédés conventionnels.

8. Formulation cosmétiques comprenant, comme ingrédient actif, un composé de formule (I) ou (II) ou un de leurs sels pharmaceutiquement acceptables, selon l'une quelconque des revendications 1 à 7, en association avec un ou plusieurs supports ou excipients physiologiquement acceptables pour cette formulation.